# EUROPEAN PATENT APPLICATION

(11) **EP 4 389 073 A1**
(43) Date of publication of application: **26.06.2024**
(21) Application number: 22894360.1
(22) Date of filing: 19.08.2022
(51) Int. Cl.: A61F 2/24

(54) **PROSTHETIC HEART VALVE**

(30) Priority: 16.11.2021 CN 202111369979
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: HU, Kaiqiang, Shanghai 201206 (CN); YANG, Wei, Shanghai 201206 (CN); WEI, Yongqiang, Shanghai 201206 (CN)
(74) Representative: Nordmeyer, Philipp Werner
(86) International application number: PCT/CN2022/113483
(87) International publication number: WO 2023/087817

(57) **Abstract**

The present invention provides a prosthetic heart valve, including a stent, prosthetic leaflets and an occlusion mechanism. Both the prosthetic leaflets and the occlusion mechanism are connected to the stent. The occlusion mechanism is able to extend outwards away from the stent and fill up a recessed portion of a native valve annulus, thereby closely fitting the prosthetic heart valve against the native valve annulus and effectively preventing paravalvular leakage.

## Description

### TECHNICAL FIELD

The present invention relates to the field of medical devices and, in particular, to a prosthetic heart valve.

### BACKGROUND

A valve in the heart with inflammation or senile calcification tends to develop, for example, calcification, fibrosis and sclerosis of the leaflets, which will compromise the leaflets' mobility. Consequently, the valve may develop stenosis or become not able to fully close. Valve replacement through surgical thoracotomy can cause great trauma to elderly patients, requires prolonged postoperative recovery and is expensive. Transcatheter heart valve treatment provides physicians with a new option, which is less traumatic and associated with fewer complications and allows faster postoperative recovery.

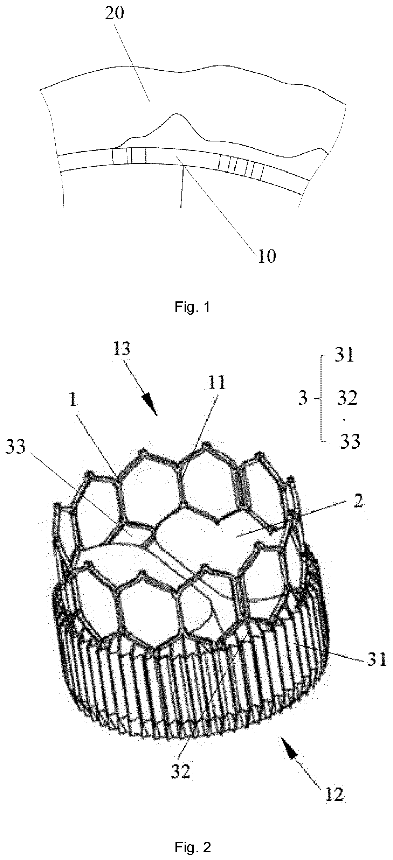

Reportedly, a large number of patients with structural heart disease have benefited from transcatheter replacement with prosthetic heart valves, which has shown year-on-year growth in use in clinical surgery. However, there are many potential complications with replacement with prosthetic heart valves, in which paravalvular leakage has been identified as one of the most common complications. Fig. 1 is a partial top view of an interface of a conventional prosthetic heart valve with a native valve annulus. As shown in Fig. 1, such commercially available prosthetic heart valves (also referred to somewhere hereinafter simply as "heart valve" for short) all rely on a scaffold 10 and an outer skirt (not shown) for being seated against the valve annulus 20. Although this can mitigate paravalvular leakage to a certain extent, since the valve annulus 20 when calcified will be irregular in shape and mostly not as round as desired, it can be difficult for the heart valve to be released so as to ensure a complete fit of the stent 10 and the outer skirt with the valve annulus 20. Consequently, one or more gaps may be left between the heart valve and the valve annulus 20, which may lead to paravalvular leakage.

### SUMMARY OF THE INVENTION

It is an objective of the present invention to solve the above-described problems with the prior art by presenting a prosthetic heart valve including an occlusion mechanism capable of filling up a recessed portion of a native valve annulus. As a result, the heart valve can be closely fitted against the native valve annulus, effectively preventing paravalvular leakage.

To this end, the present invention provides a prosthetic heart valve including a stent and prosthetic leaflets connected to the stent. The prosthetic heart valve further includes an occlusion mechanism connected to the stent, the occlusion mechanism is capable of extending outwards away from the stent.

Optionally, the occlusion mechanism may include a plurality of collapsible elements, the plurality of collapsible elements are disposed circumferentially around the stent, the plurality of collapsible elements are capable of collapse and expansion,
wherein when the collapsible elements are expanded, the collapsible elements are able to fill up a recessed portion of a native valve annulus.

Optionally, at least some of the collapsible elements may be joined together along an outer circumference of the stent.

Optionally, the occlusion mechanism may include an outer skirt disposed over at least part of an outer circumference of the stent, which is disposed outside the stent and connected thereto and is able to be caused to expand to form a plurality of collapsible elements capable of filling up a recessed portion of a native valve annulus.

Optionally, the occlusion mechanism may further include resilient elements, which are connected to the stent and configured to provide resilient forces for causing the expansion of the outer skirt.

Optionally, the occlusion mechanism may further include an inner skirt disposed over at least part of the outer circumference of the stent, which is disposed inside the stent and connected thereto in positional correspondence with the outer skirt.

Optionally, the stent may be a mesh-like hollow structure, wherein a first portion of the outer skirt is connected to struts in the stent, and a second portion of the outer skirt is connected to the inner skirt at mesh openings of the stent.

Optionally, the stent may have an inflow end and an outflow end, wherein an edge of the outer skirt corresponding to the inflow end is joined to an edge of the inner skirt corresponding to the inflow end so that the occlusion mechanism is closed at its one axial end.

Optionally, the stent may be a mesh-like hollow structure, wherein the resilient elements are disposed at intervals circumferentially around the stent so that each mesh opening of the stent circumferentially spans at least one of the resilient elements, wherein each resilient element extends in an axial direction of the stent, opposite ends of each resilient element connected to a corresponding mesh opening, and wherein the outer skirt is connected to the stent in the axial direction of the stent.

Optionally, the resilient elements may be made of a shape memory material.

Optionally, the resilient elements may be rods or tubes and may have a diameter of from 0.2 mm to 0.4 mm.

The present invention provides a prosthetic heart valve including an occlusion mechanism, which can fill up a recessed portion of a native valve annulus to closely fit the heart valve against the native valve annulus, thereby effectively preventing paravalvular leakage. Thus, good prevention and treatment of paravalvular leakage can be achieved.

In the prosthetic heart valve of the present invention, an outer skirt can be caused by resilient elements to expand to form a plurality of collapsible elements outside a stent. The prosthetic heart valve can be implanted so that the plurality of collapsible elements can automatically fill up and seal against a recessed portion of a native valve annulus, thereby achieving desirable occlusion. Moreover, the arrangement of the collapsible elements enables the prosthetic heart valve to occlude variously shaped and located recessed portions of native valve annuli of different patients. This allows the prosthetic heart valve to be used with enhanced generality in a wider range of applications.

### BRIEF DESCRIPTION OF THE DRAWINGS

Fig. 1 is a partial top view of an interface of a conventional prosthetic heart valve with a native valve annulus.
Fig. 2 is a schematic structural view of a prosthetic heart valve according to a preferred embodiment of the present invention.
Fig. 3 is a top view of the prosthetic heart valve of Fig. 2.
Fig. 4 is a schematic structural view of a stent and an inner skirt according to a preferred embodiment of the present invention.
Fig. 5 is a partial top view of an interface of a prosthetic heart valve according to a preferred embodiment of the present invention with a native valve annulus.

In these figures: 10-scaffold; 20-valve annulus; 1-stent; 11-mesh opening; 12-inflow end; 13-outflow end; 2-prosthetic leaflet; 3-collapsible element; 31-outer skirt; 32-resilient element; 33-inner skirt; 4-native valve annulus.

### DETAILED DESCRIPTION

The present invention will be described in greater detail below by way of specific embodiments thereof with reference to the accompanying drawings. From the following detailed description, features and advantages of the invention will become more apparent. Note that the figures are provided in a very simplified form not necessarily presented to scale, with the only intention of facilitating convenience and clarity in explaining the embodiments of the invention.

Directional and positional relationships described by the terms "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. are based on the orientations shown in the figures. They are intended merely to facilitate and simplify the explanation of the invention and do not indicate or imply that the stated components or elements have to assume, or be constructed or operated in, particular orientations. Therefore, they are not to be construed as limiting the invention.

As used herein, unless otherwise expressly specified or defined, the terms "mounting", "connection" and "securing" should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. As used herein, the term "plurality" means at least two, for example, two, three or more.

The present invention will be described in detail below with respect to the accompanying drawings and preferred embodiments thereof. If there is no conflict, the embodiments described below and features thereof can complement or be combined with each other.

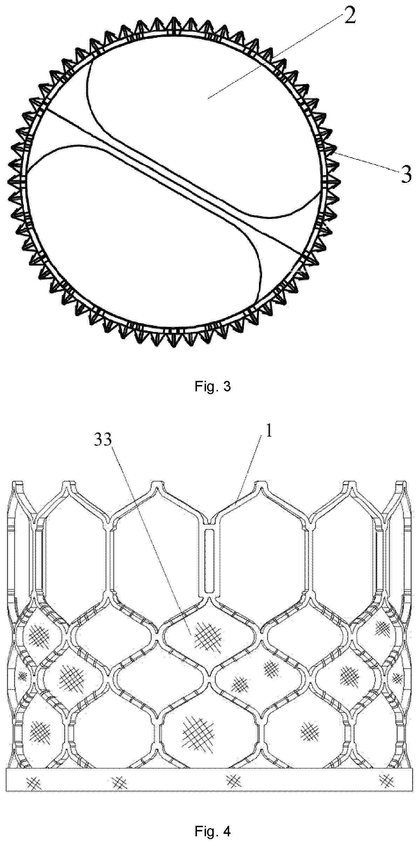

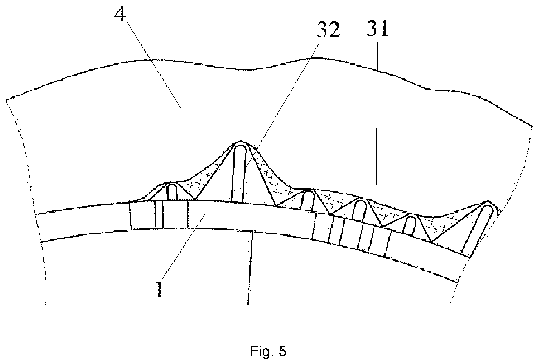

Fig. 2 is a schematic structural view of a prosthetic heart valve according to a preferred embodiment of the present invention. Fig. 3 is a top view of the prosthetic heart valve of Fig. 2. Fig. 4 is a schematic structural view of a stent and an inner skirt according to a preferred embodiment of the present invention. Fig. 5 is a partial top view of an interface of a prosthetic heart valve according to a preferred embodiment of the present invention with a native valve annulus.

As shown in Figs. 2 to 5, in a preferred embodiment of the present invention, there is provided a prosthetic heart valve for engagement with a native valve annulus 4, which includes a stent 1 and prosthetic leaflets 2. The prosthetic leaflets 2 are attached to the stent 1, and the prosthetic leaflets 2 are disposed in the stent 1.

The prosthetic heart valve further includes an occlusion mechanism attached to the stent 1, which can extend away from the stent and fit against a native valve annulus 4. As a result, the occlusion mechanism can fill up a recessed portion of the native valve annulus 4 while closely fitting against the native valve annulus 4, reducing or preventing the formation of a gap between the prosthetic heart valve and the native valve annulus 4 with the recessed portion. In this way, paravalvular leakage can be effectively prevented, providing good prevention and treatment of paravalvular leakage. Preferably, the occlusion mechanism is provided circumferentially around an outer wall of the stent 1 and can extend radially outwards from the stent 1.

It would be appreciated that the present application is not limited to any particular direction of extension of the occlusion mechanism. In alternative embodiments, the occlusion mechanism can extend in another direction than radially.

The present application is not limited to any particular structure of the occlusion mechanism, and the occlusion mechanism may be of any structure that allows it to conveniently fit against a native valve annulus 4. For example, the occlusion mechanism may be an annular structure brought into close contact with a native valve annulus 4 over the entire outer circumference of the stent 1. Alternatively, the occlusion mechanism may be a partial annular structure that fills up a recessed portion of a native valve annulus 4 over part of the outer circumference of the stent 1. Further, the occlusion mechanism may be provided inside and/or outside the stent 1.

In embodiments of the present application, the stent 1 can provide a number of functions for the prosthetic heart valve, including serving as a main body of the prosthetic heart valve and supporting the internal prosthetic leaflets 2. The present application is not limited to any particular structure of the stent 1. For example, the stent 1 may be formed by braiding or laser cutting. The present application is also not limited to any particular material of the stent 1. For example, the stent 1 may be made of an elastic material such as a nickel-titanium alloy, or a material with shape memory properties. Of course, the present application is not so limited. The stent 1 may also be formed of a plastically deformable material.

The prosthetic leaflets 2 can dynamically switch between an open configuration and a closed configuration. In the closed configuration, the prosthetic leaflets 2 sealingly abut against one another and are thereby tightly gathered or closed together. Optionally, there may be two or three prosthetic leaflets 2 (two in the example shown in Fig. 2). Compared with the design with three prosthetic leaflets 2, that with two prosthetic leaflets 2 can provide a larger effective opening area, which allows smooth flow of blood at increased blood flux.

The present invention is not limited to any particular material of the prosthetic leaflets 2, and the prosthetic leaflets 2 may be made of any suitable material or combination of materials. In some embodiments, the prosthetic leaflets 2 may be made of a biological tissue material, for example, derived from heart valve tissue of animals, or from pericardial tissue of animals, such as bovine, ovine, porcine and equine pericardial tissue. In other embodiments, the prosthetic leaflets 2 may also be made of small intestinal submucosal tissue. In other embodiments, the prosthetic leaflets 2 may also be made of a synthetic material. The synthetic material may include expanded polytetrafluoroethylene or polyester. Optionally, the synthetic material may further include one of thermoplastic polycarbonate urethane, polyether-urethane, segmented polyether-urethane, silicone-polyether-urethane, silicone-polycarbonate-urethane, ultra-high molecular weight polyethylene or a combination thereof. Preferably, the synthetic material may further include a biocompatible polymer, such as one of a polyolefin, elastomer, polyethylene glycol, polyethersulfone, polysulfone, polyvinylpyrrolidone, polyvinyl chloride, other fluoropolymer, silicone polyester, silicone polymer, silicone oligomer, silicone polylactone, or a combination or block copolymer thereof. Further, the prosthetic leaflets 2 may have experienced a surface treatment with an anticoagulant. Examples of the anticoagulant may include, but are not limited to, heparinized polymers.

In one preferred embodiment, the occlusion mechanism includes a plurality of collapsible elements 3, the plurality of collapsible elements 3 are disposed circumferentially around the stent 1 and can be collapsible and expanded. When the collapsible elements 3 are expanded, the collapsible elements 3 can fill up a recessed portion of a native valve annulus 4. With this arrangement, the collapsible elements 3 can be used to fill the recessed portion of the native valve annulus 4, such that the prosthetic heart valve can accommodate various native valve annuli 4 with recessed portions at different locations and can be applied to the native valve annuli 4 of individual patients. This allows the prosthetic heart valve to be used with enhanced generality in a wider range of applications.

It would be appreciated that the present application is not limited to how the collapse or expansion of the collapsible elements 3 is achieved. For example, the collapse and expansion may be accomplished by collapsible means disposed inside the means, or by the means themselves which are made of a shape memory material.

The present application is not limited to any particular shape of the collapsible elements 3. For example, they may be semicircular, serrated or arbitrarily otherwise shaped. Moreover, they are not limited to being regularly shaped and may be profiled. Preferably, the shape of the collapsible elements 3 is identical or similar to that of a recessed portion of a native valve annulus 4.

In some embodiments, the plurality of collapsible elements 3 are joined end-to-end and fitted and attached, as a whole, over and to the stent 1. In this way, the plurality of collapsible elements 3 can be collapsed and expanded over the entire outer circumference of the stent 1 to fill up a recessed portion of a native valve annulus 4 not matter where it is located. In other embodiments, at least some of the plurality of collapsible elements 3 are joined together over the outer circumference of the stent 1, or the plurality of collapsible elements 3 may be separate. In these cases, the shape and locations of the collapsible elements 3 over the stent 1 may be determined according to the location of a recessed portion of a native valve annulus 4 so that the collapsible elements 3 are allowed to fill up the recessed portion.

As shown in Figs. 2 and 5, in an alternative preferred embodiment, the occlusion mechanism includes an outer skirt 31, the outer skirt 31 is disposed outside of the stent 1 and attached to the stent 1. The outer skirt 31 can be caused to expand to form a number of collapsible elements 3. In this case, the collapsible elements 3 may be joined end-to-end and disposed at the location of the outer skirt 31. The collapsible elements 3 can fill up a recessed portion of a native valve annulus 4. Moreover, the outer skirt 31 can be collapsed onto the outer wall of the stent 1 to allow easier delivery, release and retrieval. In one embodiment, the outer skirt 31 is disposed over part of the outer circumference of the stent 1. In an alternative embodiment, the outer skirt 31 may be disposed over the entire outer circumference of the stent 1 (i.e., across the entire perimeter of the stent 1).

The present invention is not limited to any particular material of the outer skirt 31, and the material of the outer skirt 31 may include, without limitation, one of polyethylene terephthalate (PET), polytetrafluoroethylene (PTFE), bovine pericardial tissue and porcine pericardial tissue, or a combination thereof. The material of the outer skirt 31 is desired to have a degree of compliance, which allows the outer skirt 31 to occlude a calcified and diseased recessed portion of a native valve annulus 4 while maintaining a close fit with any non-recessed portion of the native valve annulus 4. Preferably, the outer skirt 31 is formed as a thin-wall structure, the outer skirt 31 has a thickness preferred to be in the range of 0.5 mm to 0.8 mm, which allows the collapsible elements 3 to be collapsed so that the outer skirt 31 comes into close contact with the outer wall of the stent 1 without adversely affecting the fitting and sealing of a non-recessed portion of a native valve annulus 4 with the outer wall of the stent 1, thereby ensuring efficacy of the prosthetic heart valve.

In preferred embodiments, the prosthetic heart valve further includes resilient elements 32, the resilient elements 32 are attached to the stent 1, which are configured to provide resilient forces for causing expansion of the collapsible elements 3 and hence occlusion of a recessed portion of a native valve annulus 4. The resilient elements 31 can also be collapsed, allowing the outer skirt 31 to closely fit against the outer wall of the stent 1.

In greater detail, when the outer skirt 31 is located around a non-recessed portion of a native valve annulus 4, the outer skirt 31 will be compressed by tissue of the non-recessed portion of the native valve annulus 4. Since the outer skirt 31 is rather compliant, the outer skirt 31 can closely fit against both the outer wall of the stent 1 and the tissue of the non-recessed portion of the native valve annulus 4. That is, because of its good compliance, the outer skirt 31 can be clamped between the stent 1 and the native valve annulus 4 along the circumference of the stent 1. When the outer skirt 31 is located around a recessed portion of a native valve annulus 4, the outer skirt 31 will be urged by one or more of the resilient elements 32 outwards until it closely fits on the recessed portion of the native valve annulus 4, thereby effectively reducing or preventing paravalvular leakage. It would be recognized that, as used herein, the term "outwards" refers to a direction radially away from an axis of the stent 1, and the term "inwards" refers to a direction radially towards the axis of the stent 1. It is a matter of course that the present application is not limited to causing expansion and collapse of the collapsible elements 3 using the resilient elements 32. In other instances, the expansion and collapse of the outer skirt 31 may occur due to self-expansion of the stent 1. Alternatively, a balloon may be used to cause simultaneous expansion of both the stent 1 and the outer skirt 31. Still alternatively, the outer skirt 31 itself may be made of a self-expanding material. Thus, the present application is not limited to any particular method of causing the expansion or collapse of the outer skirt 31.

Referring to Figs. 3 and 5, in this embodiment, the resilient elements 32 are each secured at one end to the stent 1 and brought into abutment at the other end with an inner side of the outer skirt 31 (as used here, the term "abutment" is defined as including both cases of being joined and not being joined). Through releasing resilient potential energy, the resilient elements 32 can provide support to the outer skirt 31 while urging it toward a recessed portion of a native valve annulus 4 so that it is expanded to form a plurality of collapsible elements 3 (each collapsible elements is in the form of a projection) distributed circumferentially around the stent. At this point, the collapsible elements 3 may define a serrated or corrugated shape. These collapsible elements 3 can be always pushed into the recessed portion regardless of the shape of the portion. Therefore, the prosthetic heart valve can be used to occlude various recessed valve annulus portions in different patients. That is, it can be used with great generality.

Preferably, the resilient elements 32 may be made of a shape memory material. For example, the resilient elements 32 may be preferably made in the form of highly resilient rods or tubes (e.g., metal rods or tubes). In this case, the rods or tubes with shape memory properties can impart good compliance to the outer skirt 31, thereby allowing the collapsible elements 3 to, once the prosthetic heart valve is implanted, automatically fill up and seal against a recessed portion of a native valve annulus 4, thereby achieving desirable occlusion. Specifically, after the prosthetic heart valve is released, when the outer skirt 31 comes into contact with a non-recessed portion of the native valve annulus 4, the resilient elements 32 are in a compressed configuration. At this point, due to their high resilience, the resilient elements 32 can release resilient potential energy that they store and thereby push the outer skirt 31 outwards radially with respect to the stent 1, thus expanding it and resulting in the formation of a plurality of collapsible elements 3 in a compressed configuration. At a result, the collapsible elements 3 can closely fit against tissue of the non-recessed portion of the native valve annulus 4. On the other hand, when the outer skirt 31 comes into contact with a recessed portion of the native valve annulus 4, the resilient rods 32 will not be subject to pressure from the native valve annulus 4 and, due to their shape memory properties, will rapidly recover their original shape, which is preferably configured so that the resilient elements 32 radiate outwards radially with respect to the stent 1. As shown in Fig. 4, as a result, the outer skirt 31 is expanded by the resilient elements 32 to form a plurality of collapsible elements 3 in an expanded configuration. The collapsible elements 3 can closely fit against tissue of the recessed portion of the native valve annulus 4. Thus, the resilient elements 32 can establish a close fit between the collapsible elements 3 and any portion (either recessed or non-recessed) of the native valve annulus 4, without leaving any gap therebetween through which paravalvular leakage may occur.

In a preferred embodiment, in order to ensure that the resilient elements 32 are as strong and plastic as desired, they are preferred to have a diameter in the range of 0.2 mm to 0.4 mm.

The present invention is not limited to any particular structure or material of the resilient elements 32, and the resilient elements 32 may be made of any material and have any structure, as long as they can provide the function of enabling collapse and expansion of the outer skirt 31. That is, the material and structure of the resilient elements 32 may be chosen as required. For example, the resilient elements 32 may be shaped and configured to define resilient features, such as springs, resilient tabs, resilient tubes, resilient rods or the like. The resilient elements 32 may be made of a resilient material, or the resilient elements 32 may be shaped and made of a resilient material additionally.

The present application is not limited to any particular number of resilient elements 32. For example, a single resilient element 32 may be used to enable collapse and expansion of one or more collapsible elements 3. Therefore, the number of resilient elements 32 may be equal to that of collapsible elements 3, or not. However, it must be ensured that, for each collapsible element 3, there is a resilient element 32 for causing its collapse and expansion. It would be appreciated that when a single resilient element 32 is used to enable collapse and expansion of multiple collapsible elements 3, it may define multiple end tines each configured to enable collapse and expansion of a respective one of the collapsible elements 3.

Referring to Fig. 4, preferably, the occlusion mechanism further includes an inner skirt 33, the inner skirt 33 is disposed inside the stent 1 and attached to the stent 1. The inner skirt 33 is in positional correspondence with the outer skirt 31 so that the stent 1 is positioned between the outer skirt 31 and the inner skirt 33 and that the collapsible elements 3 can be sealed radially. In one embodiment, the inner skirt 33 extends around part of the outer circumference of the stent 1. In an alternative embodiment, the inner skirt 33 extends around the entire outer circumference of the stent 1.

In one embodiment, the stent 1 is a mesh-like hollow structure, in which the outer skirt 31 is partially attached to struts of the stent 1, and is partially attached to the inner skirt 33 at mesh openings of the stent 1. Preferably, the outer skirt 31 is sewn to the inner skirt 33 at the openings of the stent 1. In this way, the outer skirt 31 can be caused by the resilient elements 32 to form at least one collapsible element 3 at an opening of the stent 1. Since the stent 1 is maintained between the inner skirt 33 and the outer skirt 31, the outer skirt 31 can be more firmly secured to the stent 1, effectively avoiding displacement of the stent 1 due to separation of the outer skirt 31 from the stent 1, which may lead to failure of the heart valve procedure. In an alternative embodiment, the outer skirt 31 may be attached to struts in the stent 1. In another alternative embodiment, the outer skirt 31 may be attached to the inner skirt 33.

Preferably, the occlusion mechanism is disposed at one end of the stent 1. More preferably, the stent 1 has an inflow end 12 and an outflow end 13, and the occlusion mechanism disposed at the inflow end 12 of the stent 1. In a preferred embodiment, an edge of the outer skirt 31 corresponding to the inflow end 13 of the stent 1 is joined (e.g., sewn) to an edge of the inner skirt 33 corresponding to the inflow end 13 of the stent 1 so that the occlusion mechanism is closed at one axial end. Preferably, the occlusion mechanism is closed at the inflow end 12 of the stent 1. Since the inner skirt 33 is configured so that the collapsible elements 3 can be radially sealed, during expansion of the collapsible elements 3 toward a recessed portion of a native valve annulus 4, a lumen defined by the collapsible elements 3 can be sealed both radially and at one axial end. That is, at this time, the collapsible elements 3 can define a lumen open at one axial end. After the prosthetic heart valve is implanted into the body of a patient, the collapsible elements 3 can prevent blood from flowing into the one-end-open lumen thereof or between the lumen of the collapsible elements 3 and a lumen of the stent 1. As a result, blood cannot flow into the lumen of the collapsible elements 3, preventing the closed axial end of the collapsible elements 3 from being torn open under direct impact from blood flow. Thus, recurrence of paravalvular leakage can be avoided.

Further, after the stent 1 is released, blood in the prosthetic heart valve can flow only within the lumen of the stent 1. Accordingly, the prosthetic leaflets 2 may be secured (e.g., sewn) at any arbitrary location within the lumen of the stent 1 to achieve unidirectional flow of blood. In addition, once the prosthetic heart valve is implanted to the body of a patient, blood within the lumen of the occlusion mechanism will be isolated from circulation and coagulate within the lumen of the collapsible elements 3. This can accelerate spreading and adhesion of the host's cells around the opening of the collapsible elements 3, facilitating endothelialization and the formation of a capsule there. As a result, the opening of the collapsible elements 3 can be partially closed, facilitating the prevention of paravalvular leakage. Moreover, the prosthetic heart valve can be firmly anchored to the native valve annulus 4, without any displacement relative to the native valve annulus 4. Of course, in alternative embodiments, the collapsible elements 3 may be arranged at the outflow end 13 of the stent 1, and the outer skirt 31 may be attached in the vicinity of the outflow end 13 of the stent 1. With this arrangement, the collapsible elements 3 can also define a one-end-open lumen.

Preferably, referring to Fig. 2, the stent 1 is a mesh-like hollow structure, and the resilient elements 32 are disposed circumferentially around the stent 1 at intervals in such a manner that each mesh opening 11 of the stent 1 circumferentially spans at least one of the resilient elements 32. Each resilient element 32 extends along the axis of the stent 1 and is attached at opposite ends to a corresponding mesh opening. This arrangement allows easy radial collapse and expansion of the resilient elements 32. Additionally, the outer skirt 31 may be attached to the stent 1 along the axis thereof. Specifically, portions of the outer skirt 31 may be sewn along the axis of the stent 1 through mesh openings 11 thereof to the inner skirt 33. In one embodiment, each mesh opening 11 of the stent 1 circumferentially spans a plurality of the resilient elements 32. With this arrangement, a plurality of collapsible elements 3 can be formed within each mesh opening 11 as a result of expansion of the stent 1, the collapsible elements 3 may each have a relatively small length in the circumferential direction of the stent 1. Such circumferentially shorter collapsible elements 3 can accommodate variously-shaped recessed portions of native valve annuli 4 and allows the occlusion mechanism to better seal against a native valve annulus 4, enabling more effective prevention of paravalvular leakage.

In this embodiment, referring to Figs. 2 and 3, the resilient elements 32 are axially extending metal rods (i.e., their lengthwise direction is defined along the axis of the stent), and in an expanded configuration of the metal rods, due to support from them, the outer skirt 31 may define structures in the form of triangular prisms. When the outer skirt 31 is sealed at the inflow end 12 of the stent 1, the occlusion mechanism can radially form a plurality of collapsible elements 3 defining triangular lumens, which may be distributed circumferentially around the outer wall of the stent 1. With this arrangement, radial occlusion of a recessed portion of a native valve annulus 4 can be easily achieved. The present application is not limited to any particular method of attaching the resilient elements 32 to struts of the stent 1. For example, the resilient elements 32 may be attached at opposite ends to struts of the stent 1 by suturing or welding.

Preferably, attachment holes (not shown) may be provided in the struts of the stent 1, in which opposite ends of the resilient elements 32 may be inserted and restricted in position to facilitate attachment of the resilient elements 32. In this way, the resilient elements 32 can be more securely attached to the stent 1.

In summary, the present invention provides a prosthetic heart valve including an occlusion mechanism, which can fill up a recessed portion of a native valve annulus 4 to fit the heart valve closely against the native valve annulus 4, thereby effectively preventing paravalvular leakage. Thus, good prevention and treatment of paravalvular leakage can be achieved.

In the prosthetic heart valve of the present invention, an outer skirt 31 can be caused by resilient elements 32 to expand to form a plurality of collapsible elements 3 outside a stent 1. The prosthetic heart valve can be implanted so that the plurality of collapsible elements 3 can automatically fill up and seal against a recessed portion of a native valve annulus 4, thereby achieving desirable occlusion. Moreover, the arrangement of the collapsible elements 3 enables the prosthetic heart valve to occlude variously shaped and located recessed portions of native valve annuli 4 of different patients. This allows the prosthetic heart valve to be used with enhanced generality in a wider range of applications.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A prosthetic heart valve comprising a stent and prosthetic leaflets, the prosthetic leaflets connected to the stent, wherein the prosthetic heart valve further comprises an occlusion mechanism connected to the stent, the occlusion mechanism is capable of extending outwards away from the stent.

2. The prosthetic heart valve of claim 1, wherein the occlusion mechanism comprises a plurality of collapsible elements, the plurality of collapsible elements are disposed circumferentially around the stent, the plurality of collapsible elements are capable of collapse and expansion,
wherein when the collapsible elements are expanded, the collapsible elements are able to fill up a recessed portion of a native valve annulus.

3. The prosthetic heart valve of claim 2, wherein at least some of the collapsible elements are joined together along an outer circumference of the stent.

4. The prosthetic heart valve of claim 1, wherein the occlusion mechanism comprises an outer skirt disposed over at least part of an outer circumference of the stent, the outer skirt disposed outside the stent and connected to the stent, the outer skirt able to be caused to expand to form a plurality of collapsible elements, the plurality of collapsible elements capable of filling up a recessed portion of a native valve annulus.

5. The prosthetic heart valve of claim 4, further comprising resilient elements, wherein the resilient elements are connected to the stent and configured to provide resilient forces for causing expansion of the collapsible elements.

6. The prosthetic heart valve of claim 4, wherein the occlusion mechanism further comprises an inner skirt disposed over at least part of the outer circumference of the stent, the inner skirt disposed inside the stent and connected to the stent, the inner skirt disposed in positional correspondence with the outer skirt.

7. The prosthetic heart valve of claim 6, wherein the stent is a mesh-like hollow structure, wherein a first portion of the outer skirt is connected to struts in the stent, and a second portion of the outer skirt is connected to the inner skirt at mesh openings of the stent.

8. The prosthetic heart valve of claim 6, wherein the stent has an inflow end and an outflow end, wherein an edge of the outer skirt corresponding to the inflow end is joined to an edge of the inner skirt corresponding to the inflow end so that the occlusion mechanism is closed at its one axial end.

9. The prosthetic heart valve of claim 5, wherein the stent is a mesh-like hollow structure, wherein the resilient elements are disposed at intervals circumferentially around the stent so that each mesh opening of the stent circumferentially spans at least one of the resilient elements, wherein each resilient element extends in an axial direction of the stent, opposite ends of each resilient element connected to a corresponding mesh opening, and wherein the outer skirt is connected to the stent in the axial direction of the stent.

10. The prosthetic heart valve of claim 5, wherein the resilient elements are made of a shape memory material.

11. The prosthetic heart valve of claim 5, wherein the resilient elements are rods or tubes, and the resilient elements have a diameter of from 0.2 mm to 0.4 mm.
